# EUROPEAN PATENT APPLICATION

(11) **EP 3 763 356 A1**
(43) Date of publication of application: **13.01.2021**
(21) Application number: 19186002.2
(22) Date of filing: 12.07.2019
(51) Int. Cl.: A61K 9/08, A61K 38/21, A61K 39/395, A61K 47/18

(54) **EXCIPIENT FOR BIOTHERAPEUTICS**

(71) Applicant: Ludwig-Maximilians-Universität München, 80539 München (DE); Danmarks Tekniske Universitet (DTU), 2800 Kgs. Lyngby (DK)
(72) Inventor: WINTER, Gerhard, 82377 Penzberg (DE); TOSSTORFF, Andreas, 80803 München (DE); PETERS, Günther, DK-1964 Frederiksberg C (DK)
(74) Representative: Weickmann & Weickmann PartmbB

(57) **Abstract**

The present invention relates to new excipients for stabilizing biomolecules, in particular peptides, polypeptides, nucleic acids, viruses, virus-like particles, and other types of agents. The excipients reduce aggregate and/or particle formation in preparations comprising said biomolecules and agents. The excipients are diamides of a dicarboxylic acid, in particular N,N,N',N'-tetrakis-(2-hydroxyethyl) adipinic acid amide.

## Description

The present invention relates to new excipients for stabilizing biomolecules, in particular peptides, polypeptides, nucleic acids, viruses, virus-like particles, and other types of agents, e.g. antibiotics. The excipients reduce aggregate and/or particle formation in preparations comprising said biomolecules and agents.

### Background

Peptides, polypeptides, nucleic acids, viruses, virus-like particles and other sensitive biomolecules are used frequently, e.g. as active agents in medicine, for detecting biomarkers in diagnostics [1] or as enzymes in multiple technical fields [2]. Examples of biomolecules for use in medicine include antibodies and antibody derivatives, interferons, coagulation factors such as Factor VIII, erythropoietin, interleukins, Vascular Endothelial Growth Factor, adeno-associated viruses and oncolytic herpes viruses. Examples of biomolecules for use in diagnostics include antibodies [3]. Examples of proteins and (poly)peptides for use as industrial enzymes include lipases [4] or cellulases [5].

When biomolecules are subjected to certain stress conditions like freezing/thawing, shaking, heat, shear forces and/or light, they tend to form particles or aggregates [6]. The presence of such particles or aggregates is undesired for different reasons. On the one hand, aggregation often deactivates the biomolecule, so that it does no longer fulfill its desired function [7]. On the other hand, the presence of particles or aggregates in medicaments is frequently associated with the occurrence of hazardous immune responses. In the case of pharmaceutical products, there are strict regulatory limits regarding the number of aggregates per dose [8].

Thus, it is desirable to formulate active agents, e.g. biomolecules in a way that they are stable at room temperature. In particular, they need to be resistant to shaking stress which can occur during production, handling or transport.

The stability of active agents, e.g. biomolecules, can be influenced by different parameters, e.g. the pH, the buffer substance and the ionic strength of the formulation. Further, it is possible to add excipients which help to stabilize the active agent via different mechanisms.

In principle, different types of excipients are suitable as stabilizers of biomolecules and other types of agents [9]. For example, proteins such as human serum albumin have been used frequently as stabilizers. They have, however, found to be undesirable for different reasons, since they may hamper analytics of the biomolecules to be stabilized. They are also expensive and - unless produced recombinantly - entail biological risks, for they are produced from blood. Surfactants like polysorbates or poloxamers are also often present in formulations of biomolecules. They are chemically heterogeneous, tend to have a volatile impurity profile and can oxidatively denature biomolecules [10,11]. Furthermore, it was shown in long-term studies that polysorbates disintegrate into free fatty acids which precipitate as particles [12]. As a result, surfactants are very often used as stabilizers, when they are needed to ensure stability, because no adequate alternatives could be found, but there is a very strong desire in the community of formulations scientists to get rid of polysorbates and/or to replace them by alternative stabilizers. Other commonly used excipients include amino acids, sugars and salts.

Extensive prior art regarding stabilization of biomolecules is available. However, in practice, only a few types of excipients are used. These excipients are almost exclusively selected from amino acids, sugars and sugar alcohols and surfactants. Despite decades of experience in stabilization and hundreds of publications and patents, however, stabilization of biomolecules in liquid preparations is still fraught with difficulties. Certain biomolecules cannot be sufficiently stabilized at all or they only exhibit limited stability even after freeze-drying, in particular they are only stable for a short time after reconstitution. Numerous preparations additionally exhibit particular impurities which can only be eliminated by "*bedside filtration*". Other preparations exhibit impurities, e.g. particles, nanoparticles and/or aggregates, which do not exceed the limits set by the authorities and the pharmacopoeias and are therefore being tolerated. In view of the known immunogenicity of such impurities, their quality is not optimal and should be improved.

Thus, there is an urgent need for additional new stabilizers, in particular in the field of stabilization against aggregation and/or particle formation. This is mainly because many of the available excipients have disadvantages when used alone or in combinations. For example, the effects of sugars and amino acids are mostly linked to high concentrations of such excipients. As described above, surfactants, in particular polysorbates which are most widely used, exhibit self-decomposition and catalyze decomposition of active agents and other excipients due to the presence of impurities. Dosing them, however, is difficult, since it is hard to deplete or enrich them in working steps like dialysis or filtration. They lead to foaming of solutions and intensify dissolution of leachable and extractable components from surfaces.

### Summary of the Invention

The present inventors have found that compounds from the class of dicarboxylic acid diamides are capable of stabilizing biomolecules such as antibodies or interferons in liquid preparations. In particular it was shown that when subjected to e.g. freezing/thawing stress, shaking stress or stirring stress, the presence of these substances in formulations of biomolecules leads to a reduced amount of particles compared to formulations without excipients or formulations with known excipients L-arginine or D(+)-trehalose in the same concentration. Stabilizing effects were also observed in preparations that are free from surfactants such as polysorbates.

A first aspect of the invention is the use of a diamide of a dicarboxylic acid (in the following "*diamide*'), wherein said diamide comprises at least one N-H amido group, i.e. a C(=O)-N-H group, at least one unsubstituted or substituted N-hydroxyethyl amido group, and/or at least one unsubstituted or substituted N-hydroxymethyl amido group as a stabilizer of an active agent, particularly of a biomolecule, in a liquid or dried preparation to reduce the formation of aggregates and/or particles.

A further aspect of the invention is a preparation, in particular an aqueous liquid preparation comprising an active agent, particularly a biomolecule, and a diamide of a dicarboxylic acid wherein said diamide comprises at least one N-H amido group, i.e. a C(=O)-N-H group, at least one unsubstituted or substituted N-hydroxyethyl amido group, and/or at least one unsubstituted or substituted N-hydroxymethyl amido group.

In certain embodiments, the diamide is a hydroxyalkyl diamide, i.e. a diamide comprising at least one unsubstituted or substituted N-hydroxyethyl amido group, and/or at least one unsubstituted or substituted N-hydroxymethyl amido group.

In certain embodiments, the diamide has the structure of Formula (I): 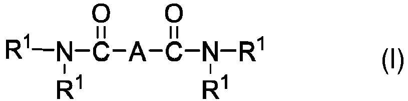
wherein each R¹ is independently selected from H and C₁-C₁₀ hydrocarbon residues, said hydrocarbon residues optionally comprising at least one heteroatom,
and wherein two R₁ together may form a ring,
with the proviso that at least one R¹ is H, a group of Formula (II) or a group of Formula (III): 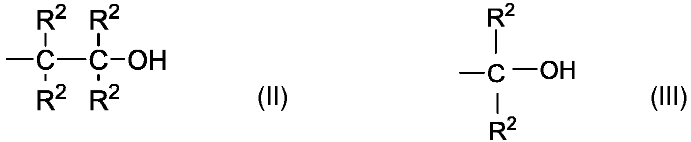
wherein each R² is independently selected from H and C₁₋₈ hydrocarbon residues, said hydrocarbon residues optionally comprising at least one heteroatom,
and
wherein A is selected from linear, branched or cyclic C₁-C₂₄ hydrocarbon residues, said hydrocarbon residues optionally comprising at least one heteroatom.

### Detailed Description of the Invention

The present invention enables the production of stable preparations of active agents, particularly biomolecules, in liquid or dried form by adding diamides as described above. The preparations can be used, for example, in industrial enzyme catalysis, in diagnostics, in cosmetics, in analytics or in medicine including human medicine and veterinary medicine. There are many more possible applications.

The terms "*stable*" and "*stabilized*" mean that preparation comprising a diamide as described above are less likely to form aggregates and/or particles when subjected to stress than formulations without an excipient. The present invention particularly stabilizes in case of freezing/thawing stress, shaking stress and/or stirring stress. It must be mentioned that such well-defined, yet somehow artificial stresses represent -as a surrogate- what a biomolecule encounters as part of the manufacturing process, regular storage time and handling and are therefore highly relevant for the quality and stability of such biomolecule and products containing such biomolecules.

*"Aggregates"* and *"particles"* as referred to in the present invention are typically in the size range of about 1 nm to about 1 mm as determined by size exclusion chromatography (SEC) and FlowCam images.

The term *"active agent"* as used herein particularly relates to biomolecules such as peptides including modified or cyclic peptides, polypeptides including unglycosylated and glycosylated, monomeric or multimeric polypeptides, nucleic acids including oligonucleotides, DNA, RNA and nucleic acid analogues, viruses, virus-like particles and other types of biomolecules, like e.g. antibiotics.

The term *"peptide"* refers to a compound comprising at least one chain of up to 50 natural or non-natural amino acids that are linked via peptide bonds. The term *"polypeptide"* refers to a compound comprising at least one chain of 51 or more natural or non-natural amino acids that are linked via peptide bonds. Peptide or polypeptide chains can be associated or linked with each other by covalent bonds and/or non-covalent interactions.

The biomolecule described herein can be, for example, a therapeutically or enzymatically active substance, or a virus vector. Non-limiting examples of biomolecules include antibodies and antibody derivatives, interferons such as interferon-alpha, interferon-beta and interferon-gamma, blood coagulation factors such as Factor VIII, erythropoietin, cytokines such as Tumor Necrosis Factor, interleukins such as interleukin 2, growth factors such as Vascular Endothelial Growth Factor, Insulin Like Growth Factor, Transforming Growth Factor (TGF), or Bone Morphogenetic Protein, as well as recombinant fusion proteins, e.g. immunoglobulin fusion proteins, enzymes such as lipases, cellulases, adeno-associated viruses or oncolytic herpes viruses. The biomolecules can further be PEGylated or glycosylated, conjugated with another active agent or they can be modified in a different way.

All biomolecules as described above are well-known and can be produced by standard methods, for example by chemical synthesis or in biological systems, e.g. cellular systems such as *E. coli,* yeast, Chinese Hamster Ovary cells or Baby Hamster Kidney cells with subsequent purification.

In a preferred embodiment of the present invention, the biomolecules are selected from antibodies including complete antibodies of different classes, e.g. IgG, IgM, IgA, IgD and IgE, modified antibodies such as single chain antibodies, antibody fragments and conjugates of such antibodies, e.g. conjugates with reporter groups, pharmaceutically active groups such as cytotoxins or radioactive groups.

In another preferred embodiment of the present invention, the biomolecules are selected from immunoglobulin fusion proteins, e.g. fusion proteins of cytokines or growth factors with constant immunoglobulin domains and conjugates of such immunoglobulin fusion proteins, e.g. conjugates with reporter groups, pharmaceutically active groups such as cytotoxins or radioactive groups.

In another preferred embodiment of the present invention, the biomolecules are selected from interferons including interferon-alpha, interferon-beta and interferon-gamma.

The term *"active agent"* as used herein also includes other types of pharmaceutically active agents such as proton pump inhibitors, e.g. omeprazole or pantoprazole, or antibiotics such as ß-lactams, macrolides, aminoglycosides, quinolones/ fluoro-chinolones, glycopeptides such as vancomycin, or tetracyclins.

The excipients of the present invention from the group of diamides are advantageous in comparison to known excipients. They are, for example, effective at relatively low concentrations. They are chemically clearly defined substances. They reduce aggregation of biomolecules and other types of active agents after different forms of stress. Particularly preferred is the use of the excipients in case of mechanic stress such as shaking stress, stirring stress, pumping stress, atomizing stress, nebulizing stress, dripping stress or dropping stress of a solution which can lead to cavitation.

The term *"diamide"* as used herein relates to a diamide of a dicarboxylic acid wherein both carboxy groups are present as carboxamide groups and wherein said diamide comprises at least one N-H amido group, i.e. a C(=O)-N-H group, at least one unsubstituted or substituted N-hydroxyethyl amido group, and/or at least one unsubstituted or substituted N-hydroxymethyl amido group.

Substituents of N-hydroxyethyl amido groups N-hydroxymethyl amido groups include C₁-C₁₀ hydrocarbon residues, particularly C₁-C₆ hydrocarbon residues and more particularly C₁-C₂ hydrocarbon residues optionally comprising at least one heteroatom, e.g. selected from halo, i.e. F, CI, Br, or I; N, O, S and/or P, particularly selected from O. The hydrocarbon residues may be selected from substituted or unsubstituted alkyl residues, wherein the term *"alkyl"* particularly includes methyl, ethyl, i-propyl, n-propyl, t-butyl, i-butyl or n-butyl. Hydrocarbon, e.g. alkyl residues may be unsubstituted or substituted by halo, e.g. F, OH, OCH₃, and/or =O.

In certain embodiments, the diamide has a solubility in water of at least about 0.02 % (w/v), of at least about 0.05 % (w/v), of at least about 1 % (w/v) or of at least about 0.5 % (w/v) at 20°C, e.g. as determined by the column elution method according to the OECD Guidelines, Test No. 105.

In certain embodiments, the diamide has a molecular weight in the range of about 120 Da to about 600 Da, e.g. about 150 Da to about 350 Da.

In certain embodiments, the diamide has both a solubility in water and a molecular weight in the ranges as indicated above.

In certain embodiments, the diamide is a compound of Formula (I) as described above.

In Formula (I) R¹ is selected from H and C₁-C₁₀ hydrocarbon residues, particularly C₁-C₆ hydrocarbon residues and more particularly C₁-C₂ hydrocarbon residues optionally comprising at least one heteroatom, e.g. selected from halo, i.e. F, Cl, Br, or I; N, O, S and/or P, particularly selected from O. The hydrocarbon residues may be selected from substituted or unsubstituted alkyl residues, wherein the term *"alkyl"* particularly includes methyl, ethyl, i-propyl, n-propyl, t-butyl, i-butyl or n-butyl. Hydrocarbon, e.g. alkyl residues may be unsubstituted or substituted by halo, particularly F, OH, OCH₃, and/or =O.

In case, two residues R¹ form a ring, this ring is typically a carbocyclic or heterocyclic 3-6 membered ring.

In specific embodiments, R1 does not contain any group which carries a charge, i.e. a positive and/or negative charge, in an aqueous solution in the pH-range of 4-9 such as a carboxylic acid group or an amino group.

In certain embodiments of Formula (I), 2, 3 or 4 of R¹ are selected from H, unsubstituted or substituted hydroxyethyl amido groups of Formula (II) and/or unsubstituted or substituted hydroxymethyl amido groups of Formula (III). In certain embodiments, 2, 3 or 4 of R¹ are selected from H. In certain embodiments, 2, 3 or 4 of R¹ are selected from groups of Formula (II). In certain embodiments, 2, 3 or 4 of R¹ are selected from groups of Formula (III).

In certain embodiments of Formula (I), all 4 of R¹ are selected from H, unsubstituted or substituted hydroxyethyl amido groups of Formula (II) and/or unsubstituted or substituted hydroxymethyl amido groups of Formula (III).

In the groups of Formula (II) and or Formula (III) each R² may be independently selected from H and C₁-C₂ hydrocarbon residues, e.g. ethyl or methyl residues, wherein said hydrocarbon residues optionally comprise at least one heteroatom, e.g. selected from halo, i.e. F, Cl, Br, or I; N, O, S, and/or P, particularly selected from O. In specific embodiments, in the groups of Formula (II) and/or Formula (III) each or at least one R² is H,-CH₃, -OH and/or =O.

In certain embodiments R¹ is selected from

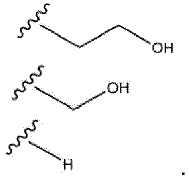

In certain embodiments, A is selected from linear or branched C₁-C₆ hydrocarbon residues or cyclic C₃-C₆ hydrocarbon residues, said hydrocarbon residues optionally comprising at least one heteroatom, e.g. selected from halo, i.e. F, CI, Br, or I; N, O, P and/or S, particularly selected from O.

In specific embodiments A is selected from

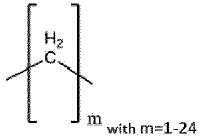

Particularly, m = 1 - 6, e.g. 1, 2, 3, 4, 5 or 6, more particularly m = 3, 4 or 5. In case m = 4, the compound is an adipinic acid diamide.

In further specific embodiments A is selected from 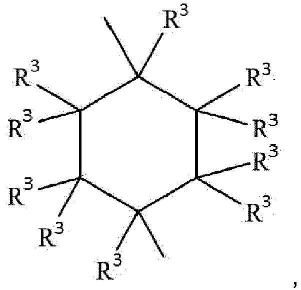 wherein each R³ is independently selected from H, OH and C₁₋₂ hydrocarbon residues, particularly from H and C₁₋₂ hydrocarbon residues wherein said hydrocarbon residues optionally comprise at least one heteroatom which may be selected from N, O, P and/or S. Particularly, a heteroatom, if present, is O. In certain embodiments, at least 8, e.g. 8, 9 or 10 residues R³ are H.

In a particular embodiment, the diamide is N,N,N',N'-tetrakis(2-hydroxyethyl) adipinic acid amide (*N*¹,*N*¹,*N*⁶,*N*⁶-tetrakis(2-hydroxyethyl)-hexanediamide-CAS# 6334-25-4):

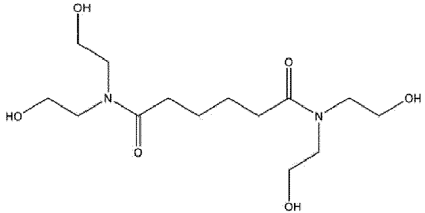

The synthesis of N,N,N',N'-tetrakis-(2-hydroxyethyl) adipinic acid amide and related compounds is well-known (see, for example, US 6 235 933 B1 and WO 2011/110624 the contents of which are herein incorporated by reference).

Other specific examples of diamides include the following compounds:
*N*¹,*N*¹,*N*⁵,*N*⁵-tetrakis(2-hydroxyethyl)-pentanediamide (CAS# 114690-06-1);
*N*¹,*N*⁶-bis(2-hydroxyethyl)-*N*¹,*N*⁶-dimethyl-hexanediamide (CAS# 57843-54-6);
*N*¹*,N*⁶-bis(2-hydroxyethyl)-*N*¹,*N*⁶-bis(2-hydroxypropyl)-hexanediamide (CAS# 1918193-23-3);
*N*¹,*N*¹,*N⁷*,*N*⁷-tetrakis(2-hydroxyethyl)-4,4-dimethyl-heptanediamide (CAS# 331862-59-0); and
*N*¹,*N*¹,*N*⁶,*N*⁶-tetrakis(2-hydroxypropyl)-hexanediamide (CAS# 57843-53-5).

In certain embodiments, the diamides can be used as stabilizers of an active agent in a preparation. The preparation can be in any physical form, for example, a liquid preparation, e.g. a solution, emulsion, suspension, or aerosol, or a solid or semisolid preparation. The diamides and preparations containing them may also be used as film coating or other kind of surface coating.

The diamide is usually added to the preparation by dissolving in an aqueous medium, but also adding it in the form of a suspension. These examples are not final or restrictive, since there are also other possible ways to combine the diamide and active agent to be stabilized.

In a specific embodiment, the preparation is a liquid preparation, particularly an aqueous preparation and more particularly an aqueous solution. In a further specific embodiment, the preparation is a liquid preparation which has been dried, for example, by freeze drying, air drying, spray drying, freeze-spray drying, or foam drying. Such a dried preparation may be reconstituted by a suitable liquid, e.g. an aqueous liquid and eventually used for its intended purpose after reconstitution.

The preparation may have any suitable pH. Typically, the pH is from about pH 4 to about pH 9 or from about pH 6 to about pH 8, e.g. about pH 7.

The preparation may further contain additional excipients such as preservatives, detergents, buffer substances or isotonicity agents.

In a specific embodiment of the present invention, the preparation does not contain a surfactant. Particularly the preparation does not contain a polysorbate, a poloxamer, solutol HS15 and/or an ionic surfactant such as SDS.

The suitable amount of diamide used in the present invention can easily be determined by the average skilled person. Typically, the concentration of the diamide in a liquid preparation may be in the range of about 1 µmol/l to about 1 mol/l, of about 100 mmol/l to about 500 mmol/l, of about 1 mmol/l to about 250 mmol/l or of about 10 mmol/l to about 100 mmol/l.

The suitable amount of active agent used in the present invention can easily be determined by the average skilled person. Typically, the concentration of the active agent in a liquid preparation may be in the range of about 0.01 mg/ml to about 300 mg/ml, of about 0.1 mg/ml to about 200 mg/ml or of about 1 mg/ml to about 150 mg/ml of active agent. In a particularly preferred embodiment, the concentration of the active agent is between 10 mg/ml and 100 mg/ml. In higher concentrations of active agent, stabilization against aggregation is very important and can also be provided by the new excipients.

The following exemplary embodiments are part of the specification:
1. Use of a diamide of a dicarboxylic acid wherein said diamide comprises at least one N-H amido group, at least one unsubstituted or substituted N-hydroxyethyl amido group and/or at least one unsubstituted or substituted N-hydroxymethyl amido group as a stabilizer of an active agent.
2. The use of item 1 wherein the diamide is a compound of Formula (I): 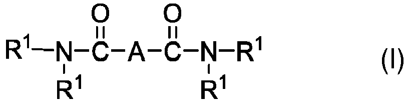
   wherein each R¹ is independently selected from H and C₁-C₁₀ hydrocarbon residues, said hydrocarbon residues optionally comprising at least one heteroatom,
   and wherein two R₁ together may form a ring,
   with the proviso that at least one R¹ is H, a group of Formula (II) or a group of Formula (III): 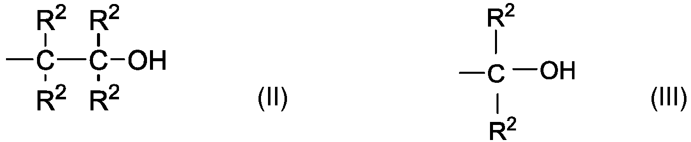
   wherein each R² is independently selected from H and C₁-₈ hydrocarbon residues, said hydrocarbon residues optionally comprising at least one heteroatom,
      and
   wherein A is selected from linear, branched or cyclic C₁-C₂₄ hydrocarbon residues, said hydrocarbon residues optionally comprising at least one heteroatom.
3. The use of item 1 or 2 wherein the active agent is stabilized in a liquid preparation, particularly in an aqueous solution.
4. The use of item 3 wherein the concentration of the active agent in the preparation is in the range from about 0.01 mg/ml to about 300 mg/ml, of about 0.1 mg/ml to about 200 mg/ml or of about 1 mg/ml to about 150 mg/ml.
5. The use of any one of items 3-4,
   wherein the concentration of the diamide in the preparation is in the range of about 1 µmol/l to about 1 mol/l, of about 100 mmol/l to about 500 mmol/l, of about 1 mmol/l to about 250 mmol/l or of about 10 mmol/l to about 100 mmol/l.
6. The use of any one of items 1-5,
   wherein the active agent is a biomolecule
7. The use of any one of items 1-6,
   wherein the active agent selected from peptides, polypeptides, nucleic acids, viruses or virus-like particles, proton pump inhibitors and antibiotics.
8. The use of item 6 or 7,
   wherein the biomolecule is selected from antibodies such as IgG antibodies, immunoglobulin fusion proteins, interferons such as interferon-2, cytokines, and enzymes.
9. The use of any one of items 1-8
   as a stabilizer in the field of medicine, cosmetics, diagnostics, and/or analytics.
10. The use of any one of items 2-9,
   wherein 2, 3 or 4 of R¹ are H, groups of Formula (II) and/or Formula (III).
11. The use of any one of items 2-10,
   wherein 2, 3 or 4 of R¹ are groups of Formula (II).
12. The use of any one of items 2-11,
   wherein in the groups of Formula (II) and/or Formula (III) each R² is independently selected from H and C₁₋₂ hydrocarbon residues, said hydrocarbon residues optionally comprising at least one heteroatom.
13. The use of item 12,
   wherein in the groups of Formula (II) and/or Formula (III) each R² is H.
14. The use of any one of items 2-13,
   wherein in the compound of Formula (I) A is selected from linear or branched C₁-C₆ hydrocarbon residues or cyclic C₃-C₆ hydrocarbon residues, said hydrocarbon residues optionally comprising at least one heteroatom.
15. The use of any one of items 1-14,
   wherein the diamide has a solubility in water of at least about 0.02 % (w/v), of at least about 0.05 % (w/v), of at least about 1 % (w/v) or of at least about 0.5 % (w/v) at 20°C.
16. The use of any one of items 1-15,
   wherein the hydroxyalkylamide is N,N,N',N'-tetrakis-(2-hydroxyethyl) adipinic acid amide.
17. The use of any one of items 1-16 as a stabilizer against aggregation and/or particle formation.
18. The use of any one of items 1-17 as a stabilizer against aggregation and/or particle formation from freezing/thawing stress, shaking stress and/or stirring stress.
19.A preparation comprising a biomolecule or a pharmaceutical agent and a hydroxyalkylamide of Formula (I): 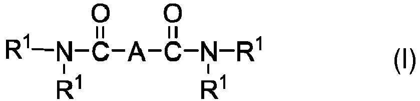
   wherein each R¹ is independently selected from H and C₁-C₁₀ hydrocarbon residues, said hydrocarbon residues optionally comprising at least one heteroatom,
   and wherein two R₁ together may form a ring,
   with the proviso that at least one R¹ is H, a group of Formula (II) or a group of Formula (III): 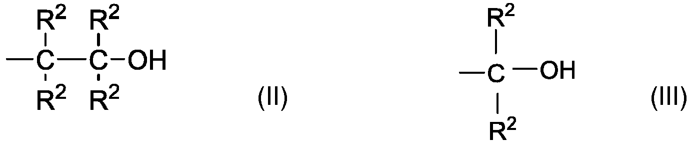
   wherein each R² is independently selected from H and C₁₋₈ hydrocarbon residues, said hydrocarbon residues optionally comprising at least one heteroatom,
      and
   wherein A is selected from linear, branched or cyclic C₁-C₂₄ hydrocarbon residues, said hydrocarbon residues optionally comprising at least one heteroatom.
20. The preparation of item 19 which is a liquid preparation, particularly an aqueous solution.
21. The preparation of item 19 or 20,
   wherein the compound of Formula (I) is defined according to any one of items 10-15.
22. The preparation of any one of items 19-21,
   wherein the compound of Formula (I) is N,N,N',N'-tetrakis-(2-hydroxyethyl) adipinic acid amide.
23. The preparation of any one of items 19-22,
   wherein the biomolecule or pharmaceutical agent is defined according to any one of items 7-8.
24. The preparation of any one of items 19-23 which has a pH from about pH 4 to about pH 9 or from about pH 6 to about pH 8.
25. The preparation of any one of items 19-24 for use in the field of medicine, cosmetics, diagnostics, and/or analytics.
26. The preparation of item 25
   for use in medicine.
27. The preparation of any one of items 19-26
   which does not contain a surfactant, particularly a surfactant selected from polysorbates, poloxamers, solutol HS15 or SDS.
28. The preparation of any one of items 19-27
   which has been dried and optionally has been reconstituted.
29. The preparation of claim 28
   which has been dried by:
   (a) freeze drying,
   (b) air drying,
   (c) spray drying,
   (d) freeze-spray drying, or
   (e) foam drying.

The present invention shall be explained in more detail by the following Examples and Figures.

### Figure Legends

**Figure 1****:** Amount of particles after 3 freezing/thawing cycles. 50 mM phosphate buffer, pH 7.0; 5 mg/ml antibody IgG trastuzumab.
**Figure 2****:** Amount of particles after stirring stress. 50 mM phosphate buffer, pH 7.0; 5 mg/ml antibody IgG trastuzumab.
**Figure 3****:** Amount of particles after 3 freezing/thawing cycles. 50 mM phosphate buffer, pH 7.0; 1 mg/ml interferon-alpha-2a.

### Examples

### Example 1: Materials and Methods

### Production of Test Solutions:

The buffer solution consisted of 50 mM sodium phosphate at pH 7.0. Protein stock solutions were liberated from other excipients by IEX chromatography to remove potential impurities and surfactants and by dialysis over 24 h in buffer solution 100-200 times of their volumes. The buffer solution was renewed after 3 hours and after 14 hours. Stock solutions of test excipients were produced by dissolving 500 mM of the excipient in 90% of the required amount of buffer solution. After that, the pH was adjusted and the remaining volume of buffer solution was added. Then, the solution was filtered with a 0.22 µm filter. The corresponding amount of protein stock solution was added. Sufficient homogenization was provided.

Freeze/Thaw-Cycle: The protein-containing solutions were filled into cleaned 2R vials and crimped. The samples were frozen from 20°C to -50°C in 3 cycles at a rate of 2K/min in a Christ 2D-6 freeze dryer and then thawed at room temperature until the entire sample had reached the liquid state, before the was cycle started again.

Stirring Stress: The protein-containing solutions were filled into cleaned 2R vials and crimped. Afterwards, the samples were stirred for 2 h at 200 rpm with a magnetic stirrer (Variomag Poly 15, Thermofisher, 3 mm polytetrafluorethylene-coated stirring bars).

Flow Imaging Microscopy: 165 µl sample solution were measured at 10x magnification using a flow imaging microscope (FlowCam, Fluid Imaging Technologies, Inc., Scarborough, ME, USA).

Size Exclusion Chromatography (Antibodies): The samples were analyzed by a Dionex Summit Chromatography system. As solid phase, a Superdex 200 Increase 10/300 GL Column (GE Healthcare) was used. The mobile phase was a 50 mM phosphate solution with 200 mM NaCl at pH 7.0. The monomer elution was detected by means of absorption of the UV signal at a wavelength of 280 nm. Recovery was defined as the proportion of the areas under the absorption curve of the monomer peak before and after stress.

Size Exclusion Chromatographie (Interferon-alpha-2a): The samples were analyzed by a Dionex Summit Chromatography system. As solid phase, a Superose 12 10/300 GL Column (GE Healthcare) was used. The mobile phase was a 50 mM phosphate solution with 200 mM NaCl at pH 7.0. The protein concentration was detected by means of UV at a wavelength of 280 nm.

### Example 2: Stabilization of an IgG antibody with N,N,N',N'-tetrakis-(2-hydroxyethyl) adipinic acid amide

N,N,N',N'-tetrakis-(2-hydroxyethyl) adipinic acid amide (Ark Pharm Inc.), L-arginine (J. T. Baker) or D(+) trehalose (Sigma-Aldrich), respectively, was added as a test excipient to an aqueous solution containing the recombinant IgG antibody trastuzumab so that the resulting solution had an IgG concentration of 5 mg/ml and contained 50 mM of excipient. The resulting solutions were subjected to freezing/thawing stress and stirring stress.

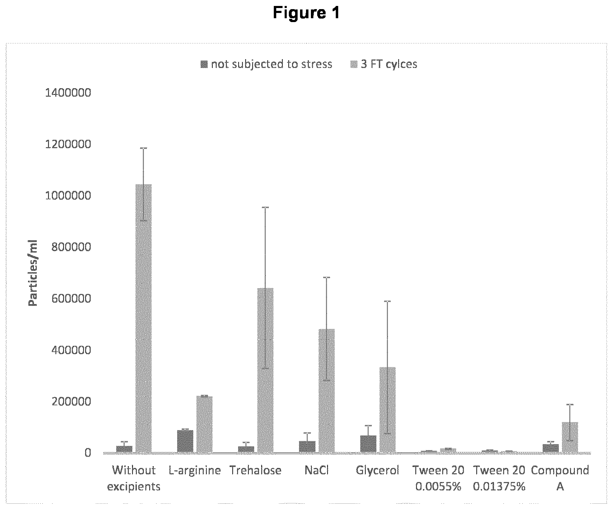

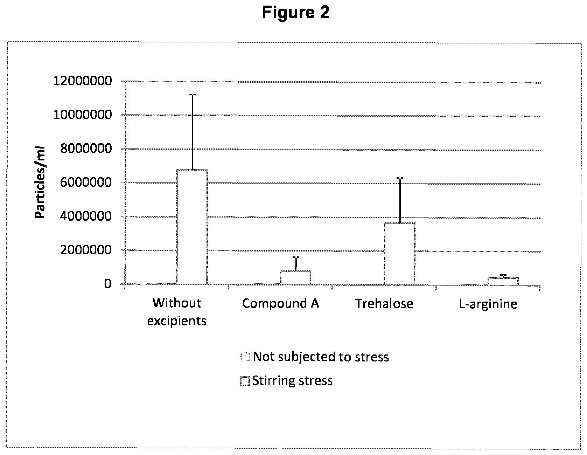

It was shown that stressed formulations comprising N,N,N',N'-tetrakis-(2-hydroxyethyl) adipinic acid amide (compound A) contain less particles than formulations comprising standard excipients like, glycerol, NaCl, or D(+) trehalose or formulations without any excipients, and similar to L-arginine. Polysorbate 20 leads to even less particles after freeze/thaw experiments, but this class of excipients is not preferred (Figures 1 and 2).

### Example 3: Stabilization of interferon-alpha-2a by N,N,N',N'-tetrakis-(2-hydroxyethyl) adipinic acid amide

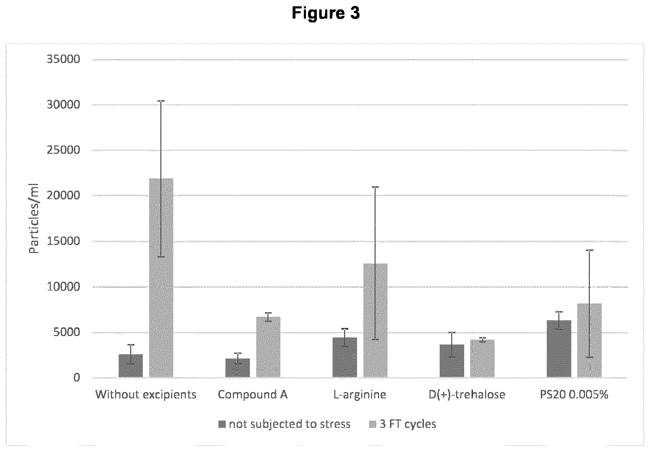

N,N,N',N'-tetrakis-(2-hydroxyethyl) adipinic acid amide was added to an aqueous solution containing interferon-alpha-2a so that the resulting solution had a protein concentration of 1 mg/ml and contained 50 mM of excipient. The resulting solutions were subjected to freeze/thawing stress. It was shown that stressed formulations comprising N,N,N',N'-tetrakis-(2-hydroxyethyl) adipinic acid amide (compound A) contain less particles than formulations without any excipient and formulations with arginine or polysorbate 20 and similar amounts as trehalose (Figure 3).

### List of references

[1] M. Dowsett, J. Bartlett, I. Ellis, J. Salter, M. Hills, E. Mallon, A. Watters, T. Cooke, C. Paish, P. Wencyk, S. Pinder, Correlation between immunohistochemistry (HercepTest) and fluorescein situ hybridization (FISH) for HER-2 in 426 breast carcinomas from 37 centres, J. Pathol. 199 (2003) 418-423. doi:10.1002/path.1313.
[2] S. Aldridge, Industry backs biocatalysis for greener manufacturing, Nat. Biotechnol. 31 (2013) 95-96. doi:10.1038/nbt0213-95.
[3] A. Lebeau, D. Deimling, C. Kaltz, A. Sendelhofert, A. Iff, B. Luthardt, M. Untch, U. Löhrs, Her- 2/neu analysis in archival tissue samples of human breast cancer: comparison of immunohistochemistry and fluorescence in situ hybridization., J. Clin. Oncol. 19 (2001) 354-63. doi:10.1200/JCO.2001.19.2.354.
[4] A. Houde, A. Kademi, D. Leblanc, Lipases and Their Industrial Applications: An Overview, Appl. Biochem. Biotechnol. 118 (2004) 155-170. doi:10.1385/ABAB:118:1-3:155.
[5] Q. Zhang, J. Bao, Industrial cellulase performance in the simultaneous saccharification and co-fermentation (SSCF) of corn stover for high-titer ethanol production, Bioresour. Bioprocess. 4 (2017) 17. doi:10.1186/s40643-017-0147-7.
[6] W. Wang, C.J. Roberts, Aggregation of Therapeutic Proteins, 2010. doi:10.1002/9780470769829.ch1.
[7] B.S. Kendrick, J.L. Cleland, X. Lam, T. Nguyen, T.W. Randolph, M.C. Manning, J.F. Carpenter, Aggregation of recombinant human interferon gamma: kinetics and structural transitions., J. Pharm. Sci. 87 (1998) 1069-1076. doi:10.1021/js9801384.
[8] United States Pharmacopeia, <788> Particulate Matter in Injections, Usp. 34 (2011)326-328.
[9] E.Y. Chi, Excipients Used in Biotechnology Products, Pharm. Excipients Prop. Funct. Appl. Res. Ind. (2016) 145-198. doi:10.1002/9781118992432.ch4.
[10] R.S.K. Kishore, S. Kiese, S. Fischer, A. Pappenberger, U. Grauschopf, H.-C. Mahler, The Degradation of Polysorbates 20 and 80 and its Potential Impact on the Stability of Biotherapeutics, Pharm. Res. 28 (2011) 1194-1210. doi:10.1007/s11095-011-0385-x.
[11] E. Ha, W. Wang, Y. John Wang, Peroxide formation in polysorbate 80 and stability, J. Pharm. Sci. 91 (2002) 2252-2264. doi:10.1002/jps.10216.
[12] A. Tomlinson, B. Demeule, B. Lin, S. Yadav, Polysorbate 20 Degradation, in: Biopharmaceutical Formulations: Quantification of Free Fatty Acids, Characterization of Particulates, and Insights into the Degradation Mechanism, Mol. Pharm. 12 (2015) 3805-3815. doi:10.1021/acs.molpharmaceut.5b00311.

## Claims

1. Use of a diamide of a dicarboxylic acid wherein said diamide comprises at least one N-H amido group, at least one unsubstituted or substituted N-hydroxyethyl amido group and/or at least one unsubstituted or substituted N-hydroxymethyl amido group as a stabilizer of an active agent.

2. The use of claim 1 wherein the diamide is a compound of Formula (I): 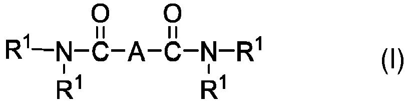
wherein each R¹ is independently selected from H and C₁-C₁₀ hydrocarbon residues, said hydrocarbon residues optionally comprising at least one heteroatom,
and wherein two R₁ together may form a ring,
with the proviso that at least one R¹, e.g. 1, 2, 3 or 4 of R¹ is H, a group of Formula (II) or a group of Formula (III): 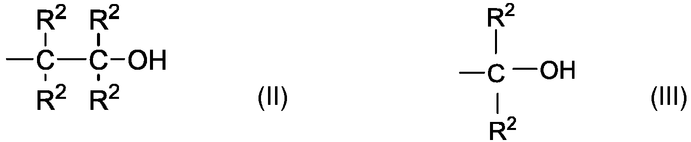
wherein each R² is independently selected from H and C₁₋₈ hydrocarbon residues, particularly from C₁-C₂ hydrocarbon residues, said hydrocarbon residues optionally comprising at least one heteroatom,
and
wherein A is selected from linear, branched or cyclic C₁-C₂₄ hydrocarbon residues, particularly from linear or branched C₁-C₆ hydrocarbon residues or cyclic C₃-C₆ residues, said hydrocarbon residues optionally comprising at least one heteroatom.

3. The use of claim 1 or 2 wherein the active agent is stabilized in a liquid preparation, particularly in an aqueous solution, particularly wherein (i) the concentration of the active agent in the preparation is in the range from about 0.01 mg/ml to about 300 mg/ml, of about 0.1 mg/ml to about 200 mg/ml or of about 1 mg/ml to about 150 mg/ml, and/or wherein (ii) the concentration of the diamide in the preparation is in the range of about 1 µmol/l to about 1 mol/l, of about 100 mmol/l to about 500 mmol/l, of about 1 mmol/l to about 250 mmol/l or of about 10 mmol/l to about 100 mmol/l.

4. The use of any one of claims 1-3,
wherein the active agent is a biomolecule, particularly selected from peptides, polypeptides, nucleic acids, viruses or virus-like particles, proton pump inhibitors and antibiotics.

5. The use of claim 4,
wherein the biomolecule is selected from antibodies such as IgG antibodies, antibody derivatives, immunoglobulin fusion proteins, interferons such as interferon-2, cytokines, and enzymes.

6. The use of any one of claims 1-5
as a stabilizer in the field of medicine, cosmetics, diagnostics, and/or analytics.

7. The use of any one of claims 1-6,
wherein the diamide has a solubility in water of at least about 0.02 % (w/v), of at least about 0.05 % (w/v), of at least about 1 % (w/v) or of at least about 0.5 % (w/v) at 20°C.

8. The use of any one of claims 1-7,
wherein the diamide is N,N,N',N'-tetrakis-(2-hydroxyethyl) adipinic acid amide.

9. The use of any one of claims 1-8 as a stabilizer against aggregation and/or particle formation, particularly as a stabilizer against aggregation and/or particle formation from freezing/thawing stress, shaking stress and/or stirring stress.

10. A preparation comprising a biomolecule or a pharmaceutical agent and a hydroxyalkylamide of Formula (I): 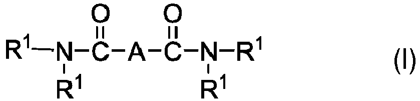
wherein each R¹ is independently selected from H and C₁-C₁₀ hydrocarbon residues, said hydrocarbon residues optionally comprising at least one heteroatom,
and wherein two R₁ together may form a ring,
with the proviso that at least one R¹ e.g. 1, 2, 3 or 4 of R¹ is H, a group of Formula (II) or a group of Formula (III): 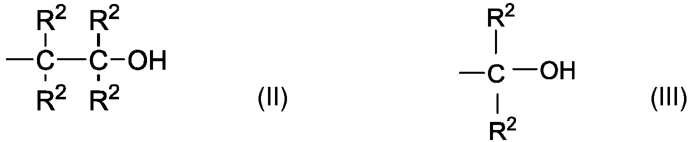
wherein each R² is independently selected from H and C₁₋₈ hydrocarbon residues, e.g. 1, 2, 3 or 4 of R¹ is H, said hydrocarbon residues optionally comprising at least one heteroatom,
and
wherein A is selected from linear, branched or cyclic C₁-C₂₄ hydrocarbon residues, particularly from linear or branched C₁-C₆ hydrocarbon residues or cyclic C₃-C₆ residues, said hydrocarbon residues optionally comprising at least one heteroatom.

11. The preparation of claim 10 which is a liquid preparation, particularly an aqueous solution.

12. The preparation of claim 10 or 11,
wherein the compound of Formula (I) is N,N,N',N'-tetrakis-(2-hydroxyethyl) adipinic acid amide.

13. The preparation of any one of claims 10-12,
wherein the active agent is defined according to any one of claims 6 or 7.

14. The preparation of any one of claims 10-13
for use in medicine.

15. The preparation of any one of claims 10-14
which does not contain a surfactant, particularly a surfactant selected from polysorbates, poloxamers, solutol HS15 or SDS.
